# EUROPEAN PATENT SPECIFICATION

(11) **EP 0 696 447 B1**
(45) Date of publication and mention of the grant of the patent: **19.01.2000**
(21) Application number: 95112564.0
(22) Date of filing: 10.08.1995
(51) Int. Cl.: A61F 2/06

(54) **Apparatus for deployment release of intraluminal prostheses**
Vorrichtung zum Positionieren einer intraluminalen Prothese
Dispositif pour la mise an place d'une prothèse intraluminale

(30) Priority: 12.08.1994 US 290021; 07.06.1995 US 475200
(43) Date of publication of application: 14.02.1996
(60) Divisional application: 99113203.6
(73) Proprietor: CARDIOVASCULAR CONCEPTS, INC., Portola Valley, CA 94028 (US)
(72) Inventor: Lenker, Jay A., Los Altos Hills California 94024 (US); Evans, Michael A., Palo Alto California 94301 (US); Kim, Steven W., Sunnyvale California 94086 (US); Glynn, Brian, Sunnyvale California 94086 (US); Watanabe, Gwendolyn A., 166 Mountain View California 94043 (US); Freislinger, Kirsten, Palo Alto California 94301 (US); Ryan, Timothy J., Los Gatos California 95032 (US); Zarins, Christopher K., Portola Valley California 94028 (US); Murphy, Richard O., Mountain View California 94043 (US)
(74) Representative: Sparing - Röhl - Henseler Patentanwälte

(56) References cited:
- EP-A- 0 274 846
- EP-A- 0 364 420
- EP-A- 0 461 791
- EP-A- 0 536 610
- EP-A- 0 596 145
- EP-A- 0 657 147
- WO-A-93/17636
- US-A- 4 793 348
- US-A- 5 078 720

## Description

### BACKGROUND OF THE INVENTION

### 1. Field of the Invention

The present invention relates generally to a delivery catheter for the endoluminal placement of resilient tubular prostheses, such as grafts, stents, stent-grafts, and other structures. More particularly, the present invention relates to a delivery catheter for the initial placing and optional repositioning of such intraluminal tubular protheses in body lumens, including blood vessels, for the treatment of abdominal and other aneurysms.

Vascular aneurysms are the result of abnormal dilation of a blood vessel, usually resulting from disease and/or genetic predisposition which can weaken the arterial wall and allow it to expand. While aneurysms can occur in any blood vessel, most occur in the aorta and peripheral arteries, with the majority of aortic aneurysms occurring in the abdominal aorta, usually beginning below the renal arteries and often extending distally into one or both of the iliac arteries.

Aortic aneurysms are most commonly treated in open surgical procedures where the diseased vessel segment is bypassed and repaired with an artificial vascular graft. While considered to be an effective surgical technique, particularly considering the alternative of a usually fatal ruptured abdominal aortic aneurysm, conventional vascular graft surgery suffers from a number of disadvantages. The surgical procedure is complex and requires experienced surgeons and well equipped surgical facilities. Even with the best surgeons and equipment, however, patients being treated frequently are elderly and weakened from cardiovascular and other diseases, reducing the number of eligible patients. Even for eligible patients prior to rupture, conventional aneurysm repair has a relatively high mortality rate, usually from 3% to 10%. Morbidity related to the conventional surgery includes myocardial infarction, renal failure, impotence, paralysis, and other conditions. Additionally, even with successful surgery, recovery takes several weeks, and often requires a lengthy hospital stay.

In order to overcome some or all of these drawbacks, endovascular graft placement for the treatment of aneurysms has been proposed. Although very promising, many of the proposed methods and apparatus suffer from other problems. In particular, delivery and placement of the endovascular graft within the vasculature can be problematic. Proper positioning and sizing of the endovascular graft is critical to the successful treatment of an aneurysm. With many endovascular graft structures and their associated delivery catheters, it is difficult or impossible to retract a partially released graft structure. Thus, improper initial placement of a vascular graft can sometimes require open surgical procedures for correction. Additionally, proper sizing of the graft can require maintenance of a large inventory of graft delivery catheters, where each catheter carries a graft having a different length and/or expansible diameter.

Furthermore, grafts are often resilient, biased to expand and anchor the graft within the body lumen. These resiliently expanding grafts are tightly compressed within the catheter and impose significant forces against the surrounding catheter bodies, often leading to excess friction between the graft and the catheter wall. These forces complicate the loading of the graft into the catheter, as well as the accurate release of grafts and stents in body lumens. Moreover, the catheters must maneuver the graft within the vascular system. Thus, the catheters are required to have flexible, elongate bodies which are particularly susceptible to the expanding graft, often resulting in invagination of the graft in the soft material of the catheter wall.

For these reasons, it would be desirable to provide an improved apparatus for endovascular placement of intraluminal protheses, including grafts, stents, and stent-grafts, for treating aneurysms and other conditions.

It would be particularly desirable to provide delivery catheters for the placement of endoluminal tubular prostheses which would facilitate the controlled release of resilient tubular prostheses. It would be particularly desirable to provide delivery catheters for the placement of endoluminal and other tubular prostheses which permit the repositioning and/or retrieval of partially released prostheses. It would be further desirable if such delivery catheters were able to contain the protheses firmly within the catheter until the final release of the prostheses into the blood vessel. It would also be particularly desirable to provide delivery catheters which reduce the frictional forces created by the resilient expansion against the catheter during loading and release of the prostheses.

### 2. Description of the Background Art

Vascular grafts and devices for their endoluminal placement are described in U.S. Patent Nos. 5,282,824; 5,242,399; 5,219,355; 5,211,658; 5,201,757; 5,192,297; 5,190,058; 5,158,548; 5,147,370; 5,104,399; 5,092,877; 5,078,726; 5,019,085; 4,990,151; 4,950,227; 4,913,141; 4,886,062; 4,820,298; 4,787,899; 4,617,932; 4,562,596; 4,577,631; and 4,140,126; and European Patent Publications 539,237; 533,511; 518,839; 518,704; 508 473; 505,686; 466 518; and 461 791. Catheters for placing vascular stents are described in U.S. Patent Nos. 5,192,297; 5,092,877; 5,089,005; 5,037,427; 4,969,890; and 4,886,062. Catheters carding a graft structure in a tube or capsule are described in U.S. Patent Nos. 5,275,622; 5,104,399; and 4,787,899; and EP466518. EP-A-364 420 describes a delivery catheter according to the preamble of independent claim 1.

### SUMMARY OF THE INVENTION

The present invention provides a delivery catheter as defined in independent claim 1. Prefered embodiments are further specified in the dependent claims.

### BRIEF DESCRIPTION OF THE DRAWINGS

Fig. 1 is a side view of a vascular graft which is exemplary of the type of radially compressible tubular prosthesis which may be placed using the delivery catheter of the present invention.
Figs. 2-18; Figs 19A-D; Figs 24A-C illustrate the use of a delivery catheter in placement of a radially compressible tubular prosthesis in a body lumen.
Figs. 20A and 20B;
Figs. 21A and 21B;

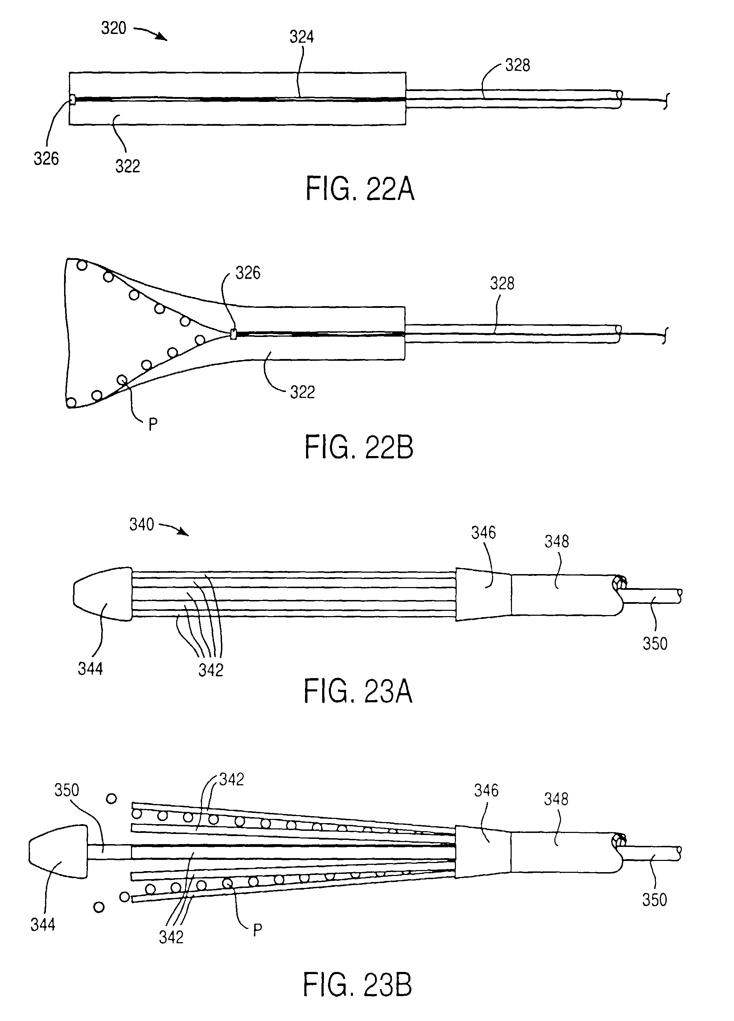

Figs. 22A and 22B; and
Figs. 23A and 23B illustrate embodiments of the retaining structure of the delivery catheter according to the invention.

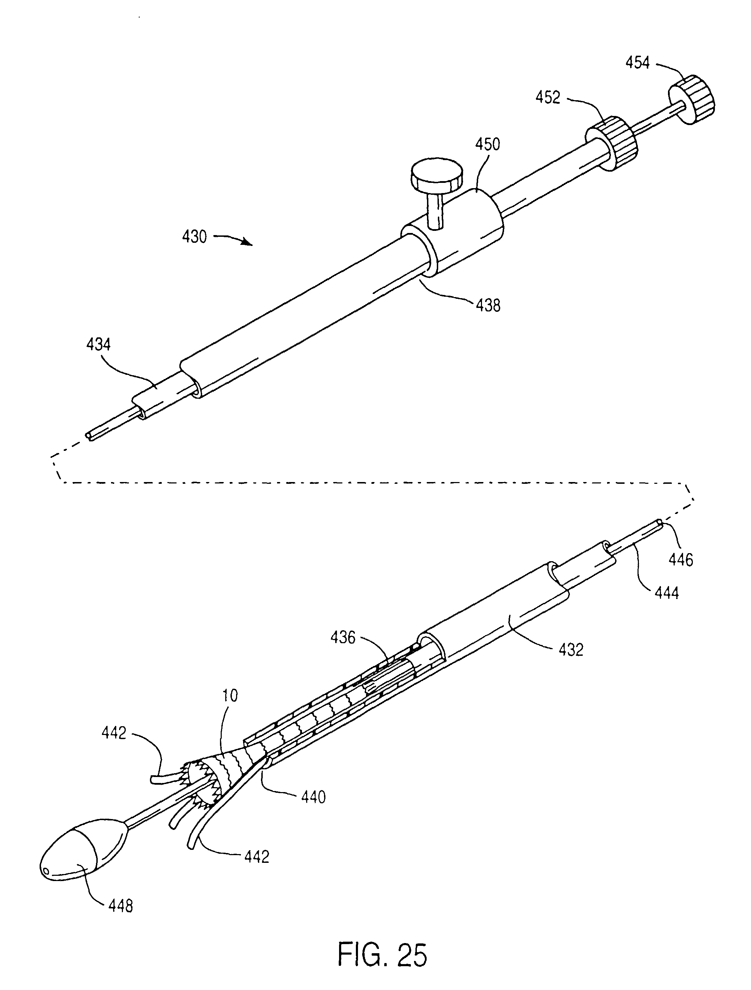

Fig. 25 is a perspective view of another embodiment of a delivery catheter of the present invention, with a portion of the distal end broken away to disclose a prosthesis therein.

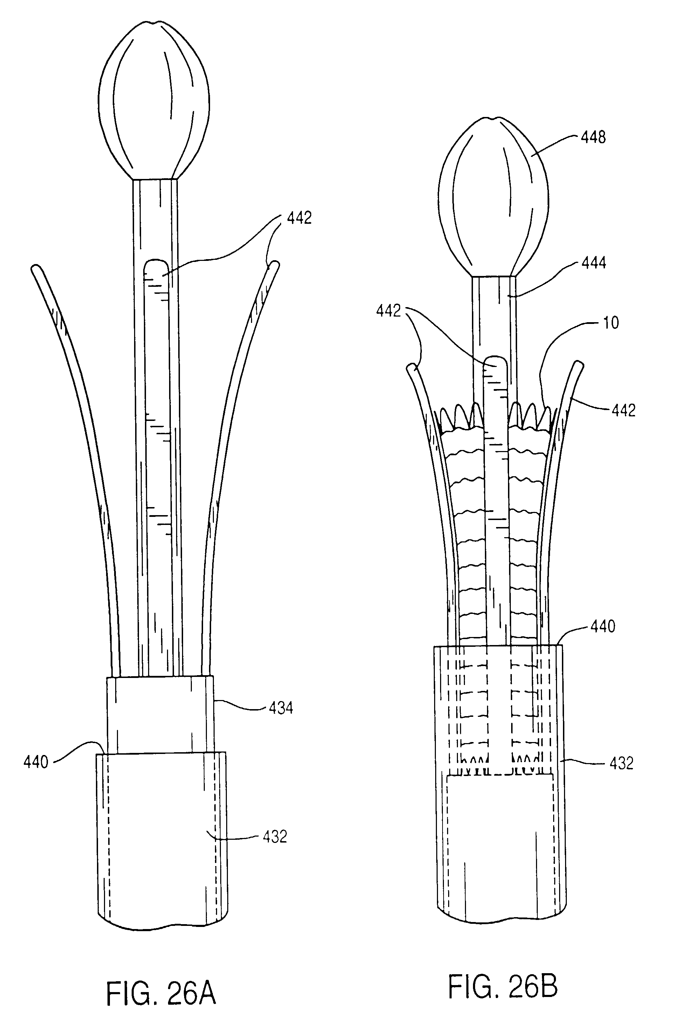

Figs. 26A and 26B illustrate the loading of a graft into the delivery catheter of Fig. 25.

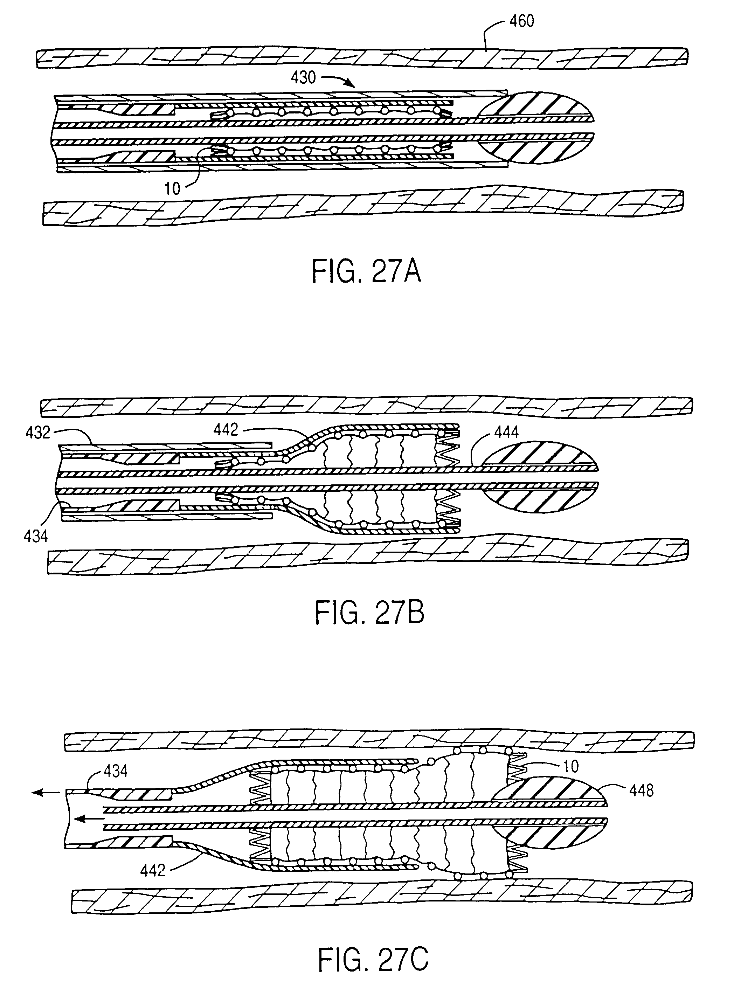

Figs. 27A-27C illustrate the use of the delivery catheter of Fig. 25 in placement of a radially compressible tubular prosthesis in a body lumen.

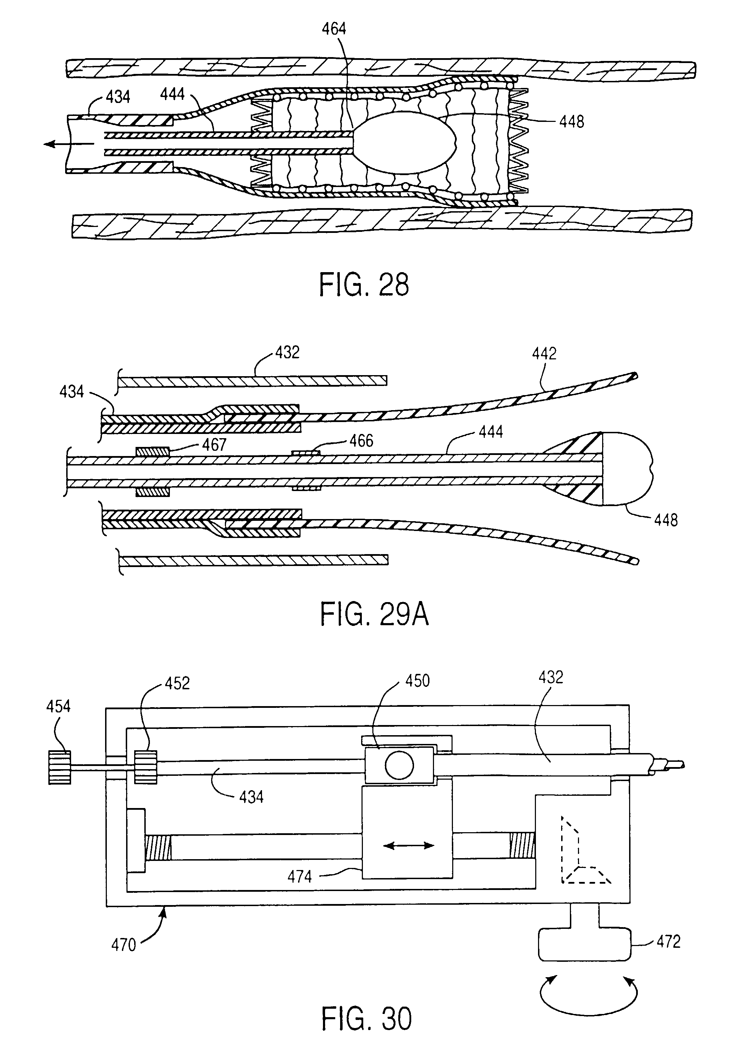

Fig. 28 illustrates a preferred method of use of the delivery catheter of Fig. 25, in which tapered nosecone is withdrawn independently of the runners.
Fig. 29A is an exploded cross-sectional view of the delivery catheter of Fig. 25.
Fig. 29B is a cross-section of an alternative shaft structure and cover having increased flexibility.
Fig. 30 illustrates a housing at the proximal end of the delivery catheter of Fig. 25 which provides a mechanical advantage for withdrawing the cover.

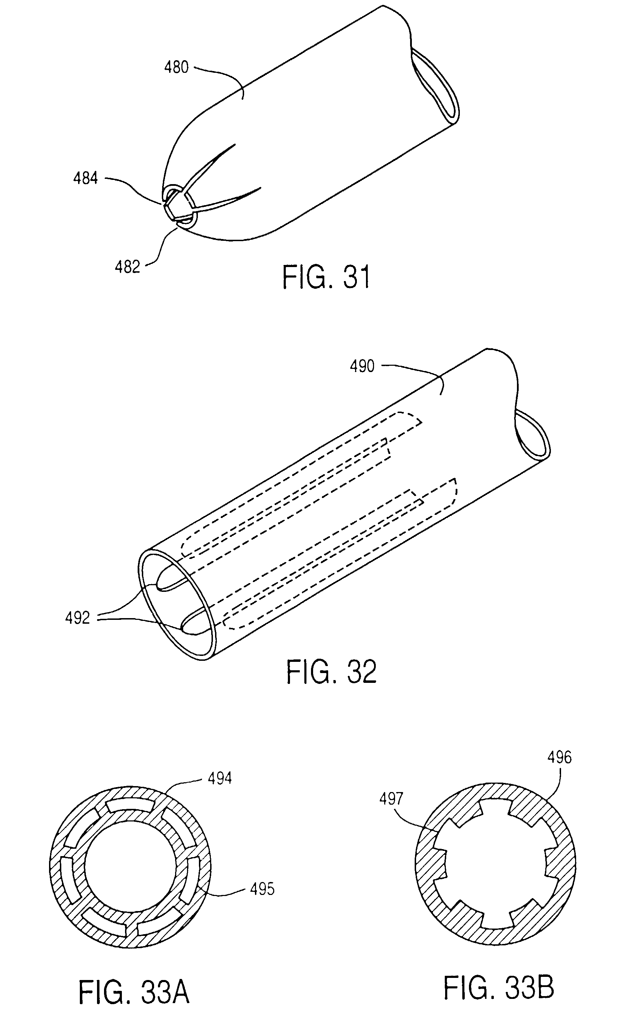

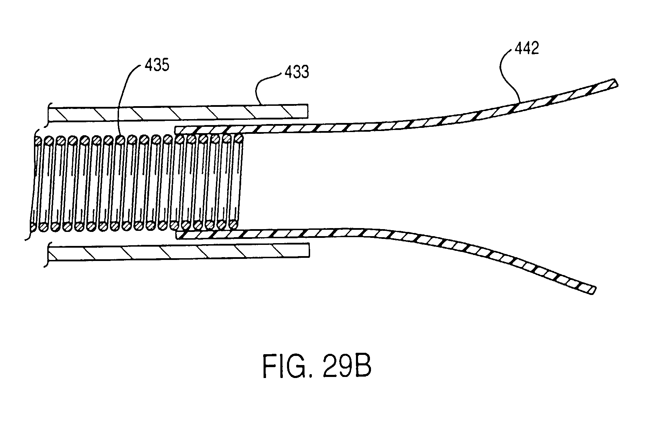

Fig. 31 illustrates a delivery catheter cover having a rounded, atraumatic distal end with a split tip.
Fig. 32 illustrates a delivery catheter cover having runners imbedded within the distal end.
Figs. 33A and 33B are alternative cross-sectional views of a delivery catheter cover.

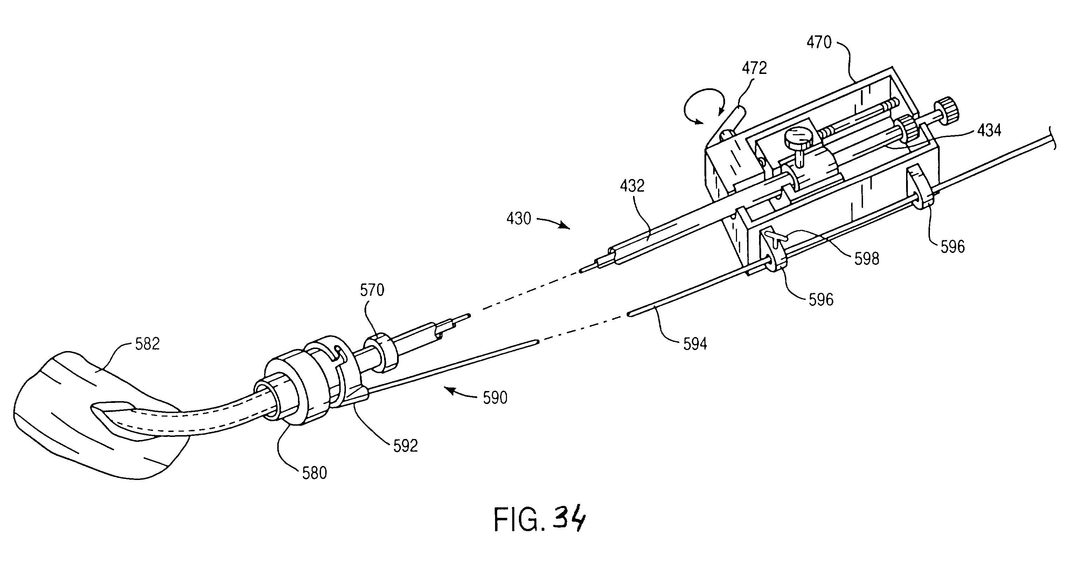

Fig. 34 illustrates a brace which restrains the prosthesis at a target location during deployment.

### DESCRIPTION OF THE PREFERRED EMBODIMENT

The present invention provides a delivery catheter as defined in claim 1 for the endoluminal placement of intraluminal tubular prostheses, particularly grafts, stents, and stent-grafts. The tubular prostheses will be radially compressible, and the apparatus of the present invention will maintain the prostheses under compression in a narrow-diameter configuration while they are being introduced to the body lumen, typically during surgical cutdown or percutaneous introduction procedures. Placement of the tubular prosthesis is effected by releasing the prosthesis at a target location in the lumen. Thus, it is necessary that the prosthesis be sufficiently resilient and conformable to expand against the interior wall of the body lumen. It will be appreciated, however, that the prosthesis may be formed at least partly from malleable components which permit it to be subsequently further expanded, typically by inflation of a balloon within the lumen of the prosthesis.

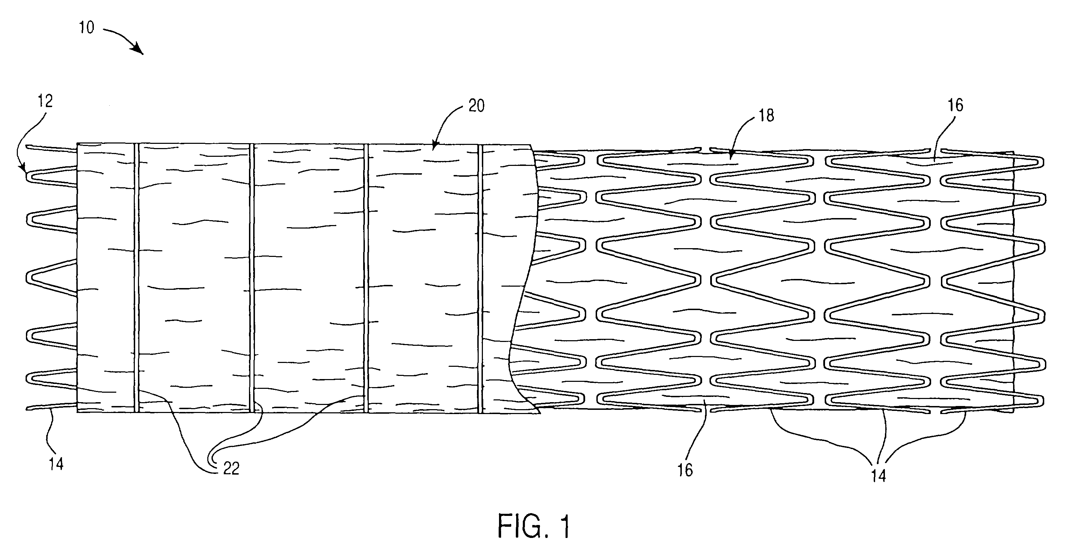

The present invention will find greatest use in the percutaneous placement of endovascular prostheses for the treatment of diseases of the vasculature, particularly aneurysms, stenoses, and the like. Suitable prosthesis structures which may be deployed by the delivery catheter of the present invention are described in copending application Serial No. 08/255,681. One exemplary graft structure 10 is illustrated in Fig. 1. Prosthesis 10 comprises a perforate tubular frame 12 which includes a plurality of independent (non-connected) band members 14 separated from each other by small gaps 16. The tubular frame 12 is covered by an inner liner 18 and an outer liner 20, where the inner and outer liners together encase or sandwich the otherwise free-floating band members 14 therebetween. In order to secure the band members 14 in place, and secure the liners to the perforate tubular frame 12, the inner and outer liners are joined together along circumferential lines 22, preferably aligned with the gaps 16 between adjacent band members 14. The liners may be joined together by stitching, heat welding, ultrasonic welding, or the like. In the exemplary embodiment, the liners 18 and 20 are formed from polymeric sheet material and are joined together by ultrasonic welding. The band members 14 at each end of the graft 10 will have to be further secured to the liners 18 and 20. For example, they could be stitched, welded, or otherwise joined to the liners to hold them in place. The graft 10 will typically have a length in the range from about 50 mm to 500 mm, preferably from 80 mm to 200 mm, with a relaxed diameter in the range from about 4 mm to 45 mm, preferably being in the range from 5 mm to 25 mm. Such graft structures will be particularly suitable for treating vascular aneurysms.

In connection with the present invention, it has been discovered that the placement of resilient tubular prostheses imposes serious demands on delivery and imaging systems, as well as on the attending medical personnel. Prostheses are highly compressed within delivery catheters to allow maneuvering within the vascular system. The compressive forces have been found to lead to excessive friction during deployment from the delivery catheters of the prior art. Additionally, visualization of compressed prostheses within the catheter is problematic, particularly when a branched prosthesis must be placed in a branching body lumen in a specific orientation.

The delivery catheters facilitate deployment of resilient prostheses by reducing friction at the prosthesis/catheter interface, avoiding any increase in the stiffness of the delivery system where it is not needed. It has been discovered that compressed prostheses are largely rigid, which reduces any penalty in flexibility imposed by including hard, friction-reducing runners around the prosthesis.

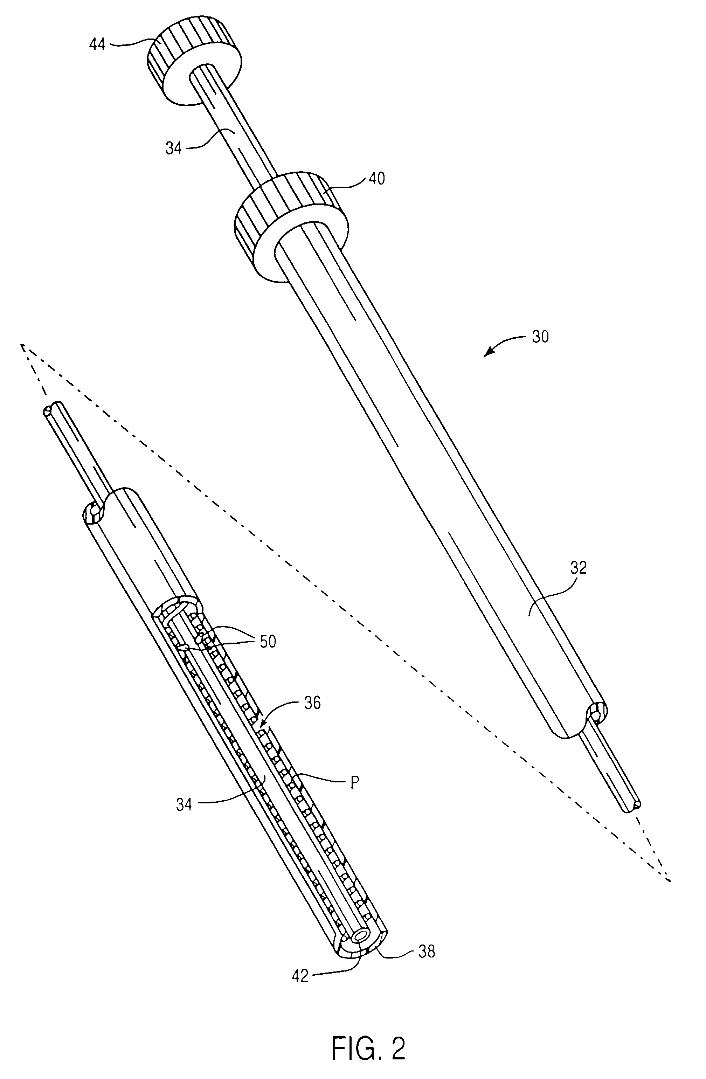

Referring now to Fig. 2, a delivery catheter 30 comprises a sheath 32 and a shaft or inner catheter body 34. The sheath 32 has a central lumen 36 extending from a distal end 38 to a proximal handle 40. The shaft 34 is slidably received within the central lumen 36 and has a distal end 42 and a proximal handle 44. The delivery catheter 30 receives a radially compressible tubular prosthesis P within the annular space between the outer surface of the shaft 34 and the inner surface of the lumen through sheath 32. For convenience, the prosthesis is illustrated as a radially compressed helical coil which expands by unwinding and axial shortening. The delivery catheters, however, can be used with virtually any radially compressible prosthesis, as described above.

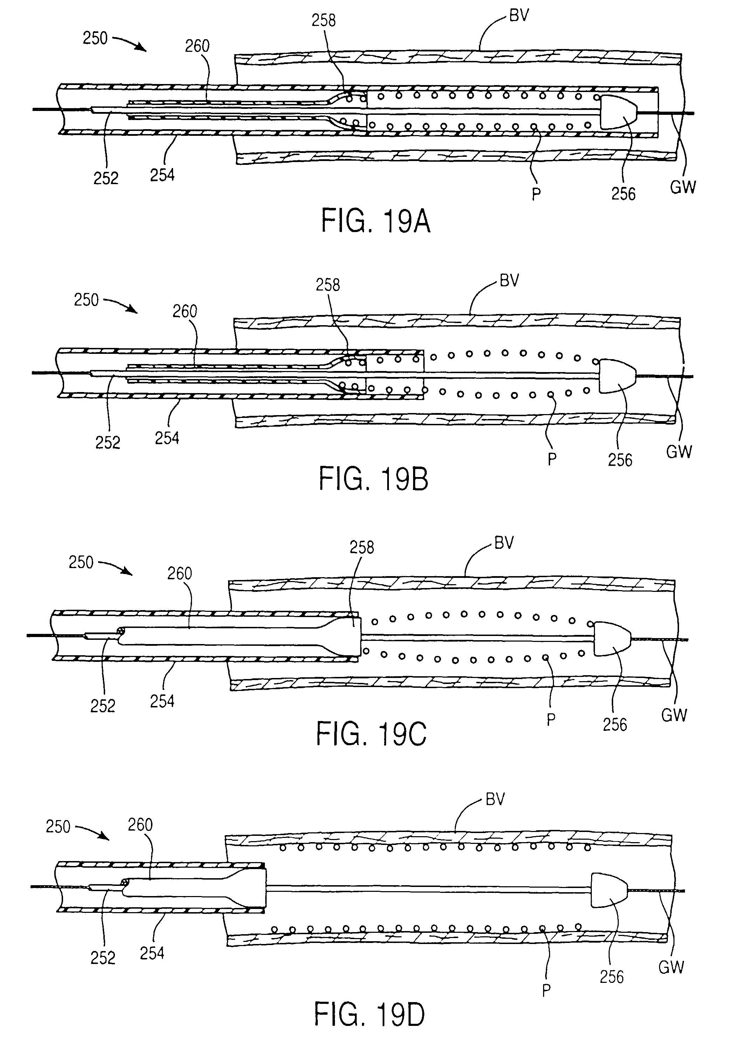

The delivery catheter of Fig. 2 relies on maintaining the radial compression of prosthesis P by direct pressure from the sheath 32. As will be discussed in detail below in connection with Figs. 19-24, prosthesis compression may also be provided by a retaining structure which comprises a cover, spaced-apart anchors, or other equivalent structure which maintains the radial compression regardless of the position of the sheath. Using such embodiments, the prosthesis may be uncovered and located prior to release and radial expansion.

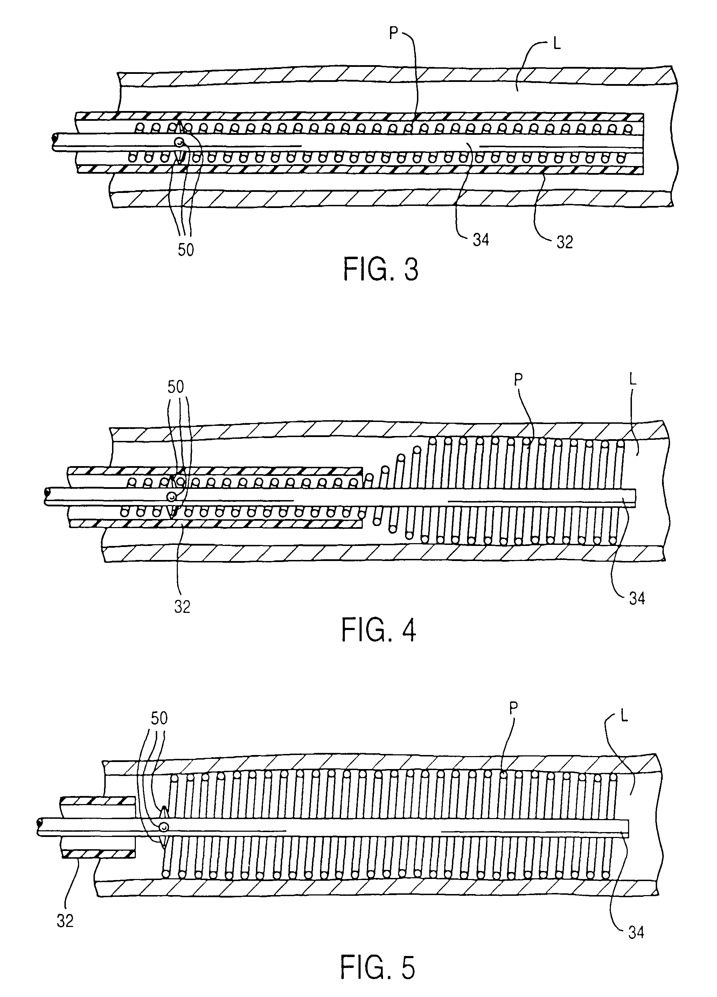

In the embodiment of Fig. 2, the prosthesis P is anchored by a plurality of penetrating stay members 50 which are circumferentially spaced-apart over the exterior of the shaft 34. The stays 50 will be spaced proximally from the distal end 42 of the shaft 34 by a distance which corresponds generally to that of the tubular prosthesis P which is to be maintained on the delivery catheter 30. The penetrating stays 50 will extend radially outward by a distance sufficient to engage the interior surface of the lumen 36 of the sheath 32. In that way, the penetrating stays 50 will be able to anchor the proximal end of the tubular prothesis P when it is held within the catheter. In particular, the prosthesis P will remain anchored as the sheath 32 is drawn proximally over the shaft 34, as illustrated in Figs. 3-5.

When initially placed in a body lumen L, the sheath 32 covers substantially the entire length of the prosthesis P with the penetrating stays 50 engaging the proximal portion of the prosthesis P, as illustrated in Fig. 3. The sheath 32 may then be retracted proximally, partially releasing the prosthesis P, as illustrated in Fig. 4. The proximal portion of the prosthesis P, however, remains anchored by the penetrating stays 50 so long as the sheath 32 remains positioned over the stays. Once the sheath 32 is withdrawn to the proximal side of the stays 50, as illustrated in Fig. 5, the prosthesis P will be fully released. Prior to such full release, however, the prosthesis P may be recaptured by advancing the sheath 32 in the distal direction relative to the shaft 32.

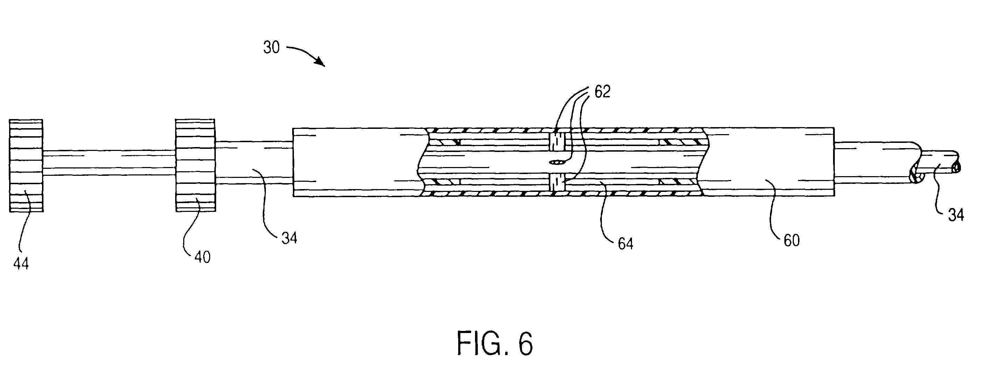

Referring now to Fig. 6, the catheter 30 may optionally be provided with a journal sleeve 60 near its proximal end. The journal sleeve 60 is preferably mechanically coupled to the shaft 34 by pins 62 which extend through slots 64 in the sheath 32. The journal sleeve 60 can be anchored within an introducer sleeve or other access device (not illustrated) which is used to provide percutaneous access to the body lumen being treated. After initial positioning of the catheter 30 so that the prosthesis P is located at the target location within the lumen, it is desirable to firmly anchor the catheter 30 within the introducer sheath. Journal sleeve 60 permits anchoring of the shaft 34 (which carries the prosthesis P) while allowing the sheath 34 to remain freely translatable relative to both the journal sleeve 60 and the catheter shaft 34.

The dimensions and materials of construction of the catheter 30 may vary widely, depending on the intended usage. For vascular applications, the catheter 30 will typically have a length in the range from about 50 cm to 250 cm, preferably from 100 cm to 200 cm, and a diameter in the length from about 3 mm to 8 mm, preferably from 4 mm to 6 mm. These dimensions generally refer to the exterior dimensions of the sheath 32. It will be appreciated that the catheter shaft 34 will have a smaller diameter, typically in the range from 1 mm to 5 mm, preferably from about 1.5 mm to 3 mm, allowing a sufficient annular space therebetween to receive the prosthesis P. The catheter shaft will also have a length which is greater than that of the sheath, usually by a distance sufficient to accommodate the length of the prosthesis which is being delivered, typically from 5 cm to 25 cm, preferably from 7.5 cm to 15 cm. The catheters will generally be constructed of natural or synthetic polymers, such as silicone rubber, natural rubber, polyvinylchloride, polyurethanes, polyesters, polyethylenes, polytetrafluoro-ethylenes (PTFE), and the like. Optionally, the catheter sheath and shaft may be formed as composites having a reinforcement layer incorporated within a polymeric body in order to enhance strength, flexibility, and toughness. Suitable reinforcement layers include wire mesh layers, braided layers, and the like. The tubular members of the present invention may be formed by extrusion, with the tubular diameter modified by heat expansion and/or shrinkage using conventional techniques. Particular techniques for forming vascular and other catheters suitable for use in the present invention are well described in the patent and medical literature.

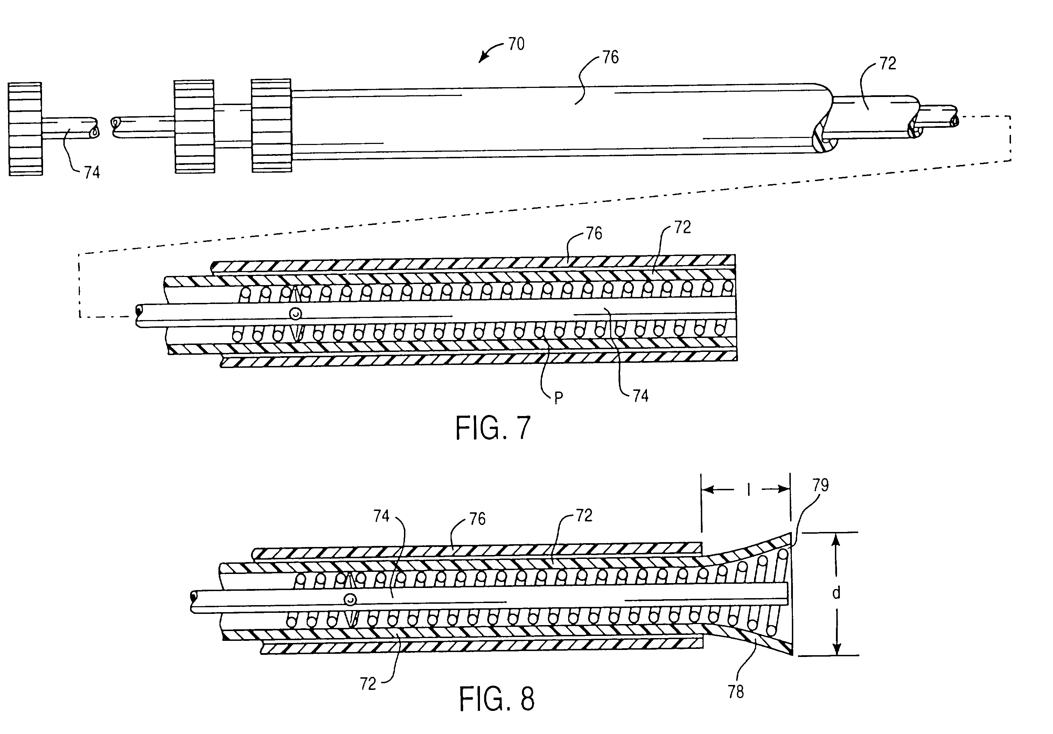

Referring now to Figs. 7 and 8, a catheter 70 having a sheath 72 with a deployable flared end will be described. Catheter 70 comprises the sheath 72, a shaft 74, and a prosthesis-containment sheath 76. A prosthesis P is contained between the sheath 72 and the shaft 74, generally as described above in connection with delivery catheter 30. The sheath 72, however, differs from that of sheath 32 in that sheath 72 has an outwardly flared distal end 78, as best seen in Fig. 8. The distal end 78 is a resilient structure, typically formed from the material of the sheath 72 itself and optionally having a plurality of elastic reinforcement elements imbedded therein to maintain the desired flared configuration, and may be radially collapsed by the containment sleeve 76, as illustrated in Fig. 7. The flared distal end of the sheath 72 is advantageous since it facilitates the release and recapture of the prosthesis P.

The flared distal end 78 of catheter 70 will usually have a fully expanded diameter d at the distal tip 79 in the range from 10 mm to 30 mm, preferably from 15 mm to 25 mm. The distal tip of diameter d will usually be greater than the diameter of the proximal portions of the sheath 72 by a factor from 2 to 8, preferably being from 2.5 to 5. The flare will extend over an axial length ℓ in the range from 3 mm to 30 mm, preferably from 5 mm to 20 mm. These flare dimensions will generally be applicable to all embodiments of the present invention where the prosthesis-containment sheath has a flared distal end.

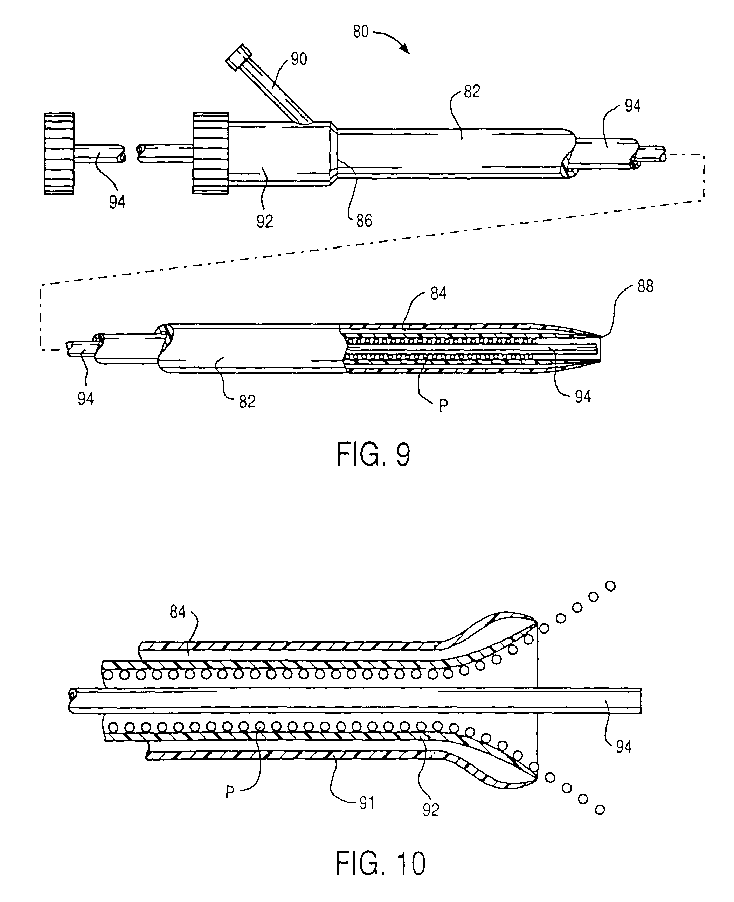

Referring now to Figs. 9 and 10, a catheter 80 having an alternate mechanism for deploying a flared distal tip on a prosthesis-containing sheath structure 82 will be described. Catheter 80 comprises the sheath 82 having an annular lumen 84 extending from its proximal end 86 to its distal end 88. The annular lumen 84 is connected to an inflation port 90 on a proximal housing 92. A shaft 94 extends through the central lumen of the sheath 82 and carries a prosthesis P near it distal end.

The distal end of the sheath 82 is formed so that, upon inflation with a non-compressible fluid medium, typically saline or other biocompatible liquid, it assumes the outwardly flared configuration shown in Fig. 10. The structure is sufficiently elastic, however, so that removal of the inflation medium will permit the sheath 82 to resume its non-flared configuration, as illustrated in Fig. 9. Flaring of the distal end of sheath 82 facilitates both release and recapture of the prosthesis P, as with the embodiment of Figs. 7 and 8.

Conveniently, distal end 88 of sheath structure 82 comprises an outer layer 91 secured to an inner layer 92 at their respective distal ends. Both layers 91 and 92 will be composed of a flexible, non-distendable material, such as polyethylene terephthalate (PET), or other reinforced material, such as an elastomeric or non-elastomeric material reinforced with a non-distendable mesh. The outer layer will be shorter than the inner layer so that when the annular lumen 84 is inflated, the distal end will flare as shown in Fig. 10.

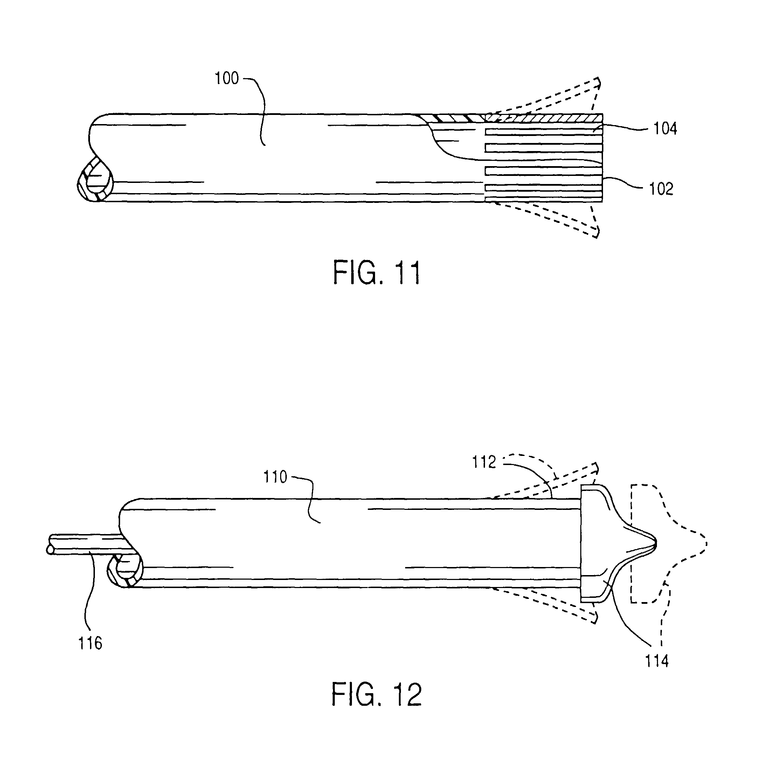

Alternative mechanisms for providing a deployable flare at the distal end of a prosthesis-containment sheath are illustrated in Figs. 11 and 12. The sheath 100 in Fig. 11 has a distal end 102 including a plurality of axially aligned, circumferentially spaced-apart heat memory alloy members 104. The heat memory alloys are selected to have a temperature transition where they assume a straight, non-flared configuration at low temperatures, as illustrated in full line in Fig. 11. At body temperature, however, the members 104 assume an outwardly flared configuration, as illustrated in broken line. Suitable alloy materials include nickel-titanium alloys which may be heat treated to provide the proper shapes and transition temperature.

Sheath 110 illustrated in Fig. 12 has a resilient, flared structure formed at its distal end 112. The flared distal end 112 is contained in an end cap 114 which may be distally advanced (as illustrated in broken line) by shaft 116 to release the flared end structure, as shown in broken line.

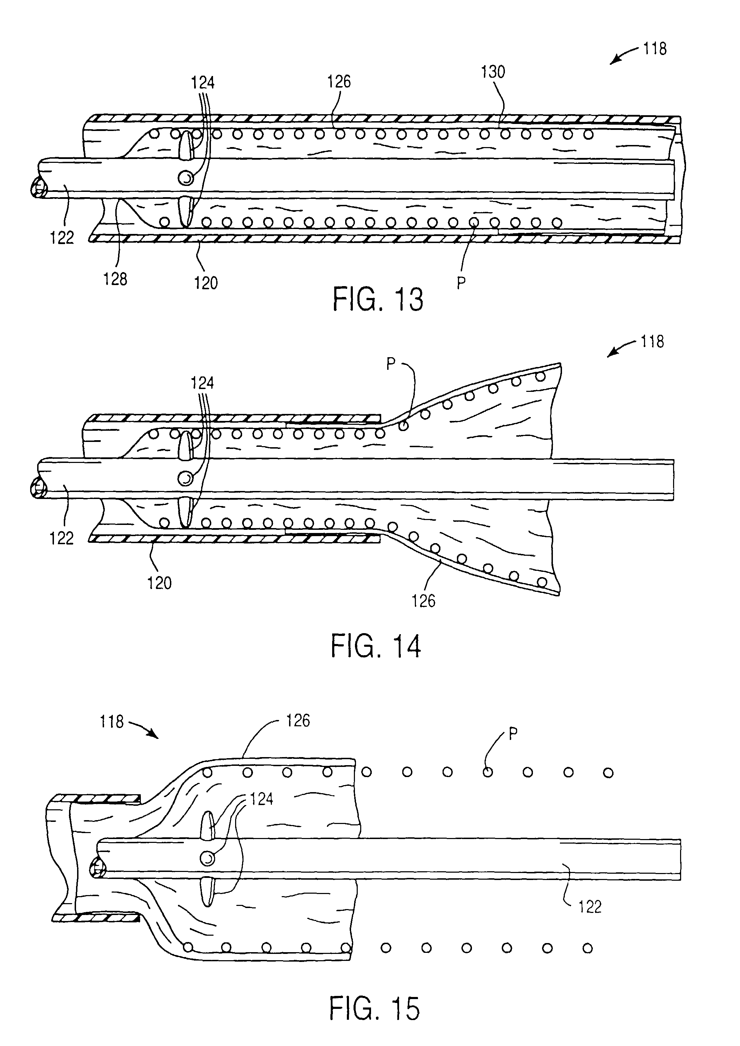

An alternative structure for facilitating the release and recapture of a prosthesis from a delivery catheter is illustrated in Figs. 13-15. A catheter 118 is provided with a sheath 120, shaft 122, and penetrating stays 124, generally as described above in connection with Figs. 2-5.

The catheter 118 further includes an eversible membrane 126 which is attached at a first end 128 to the shaft 122, and at a second end 130 to the inner surface of the lumen of sheath 120. The membrane 126 will be formed from a flexible, preferably lubricous and non-compliant material, such as PET, nylon, polytetrafluoroethylene (PTFE), any of which may be wire- or braid-reinforced, or the like. The prosthesis P will remain anchored on the shaft 122 by penetrating stays 124 as the sheath 120 is partially withdrawn (Fig. 14). The membrane 126 folds back over itself (everts) as the sheath 120 is retracted so that there are always two layers of the membrane between the distal end of the sheath and the prosthesis P. The double-layer structure of the membrane provides a high degree of lubricity during the release and optional recapture of the prosthesis P. Complete release of the prosthesis P is illustrated in Fig. 15.

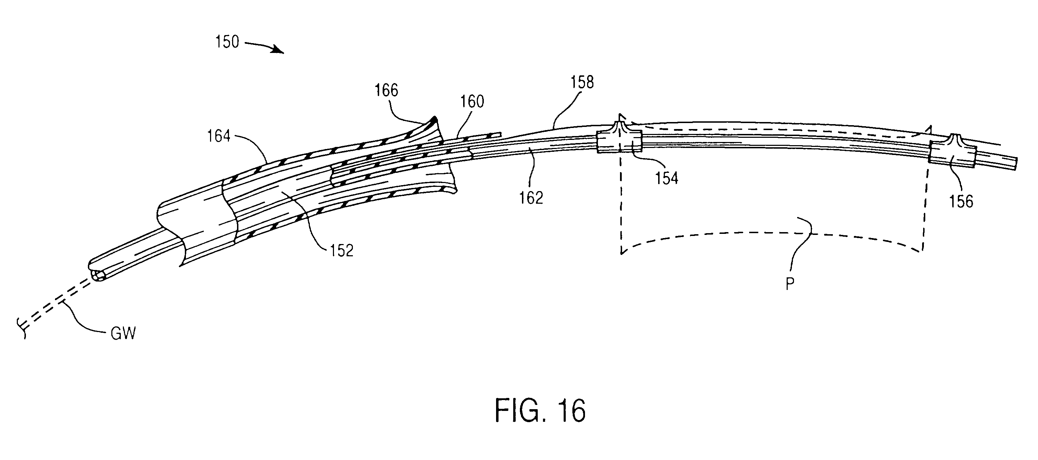

Referring now to Fig. 16, an alternative prosthesis anchoring mechanism for a delivery catheter 150 is illustrated. The delivery catheter 150 includes a shaft 152 having a pair of axially spaced-apart stays 154 and 156. A pull wire 158 extends through a lumen 160 of shaft 152 and through protrusions on each of the stays 154 and 156. A guide wire GW is received through the shaft 152 in order to permit vascular introduction by conventional techniques. The radially compressible prosthesis P (such as graft 10) is placed over the distal end of the shaft extension 162, generally being aligned between the stays 154 and 156. The pull wire 158 is then advanced through the stays 154 and 156 so that it passes through each end of the prosthesis P to maintain the prosthesis P in place until the pull wire is withdrawn. While the pull wire 158 remains in place, a prosthesis-containment sheath 164 may be axially advanced over the graft to radially compress the graft into its desired low profile diameter. The sheath 164 includes a flared (i.e., outwardly tapered) distal end 166 to facilitate advancing the sheath over the prosthesis P, in particular so that the prosthesis P may be recaptured when it is partially deployed. The outward flare may be permanently fixed in the body of the sheath, but will preferably be selectively deployable between the flared and non-flared configuration, using any of the mechanisms described above.

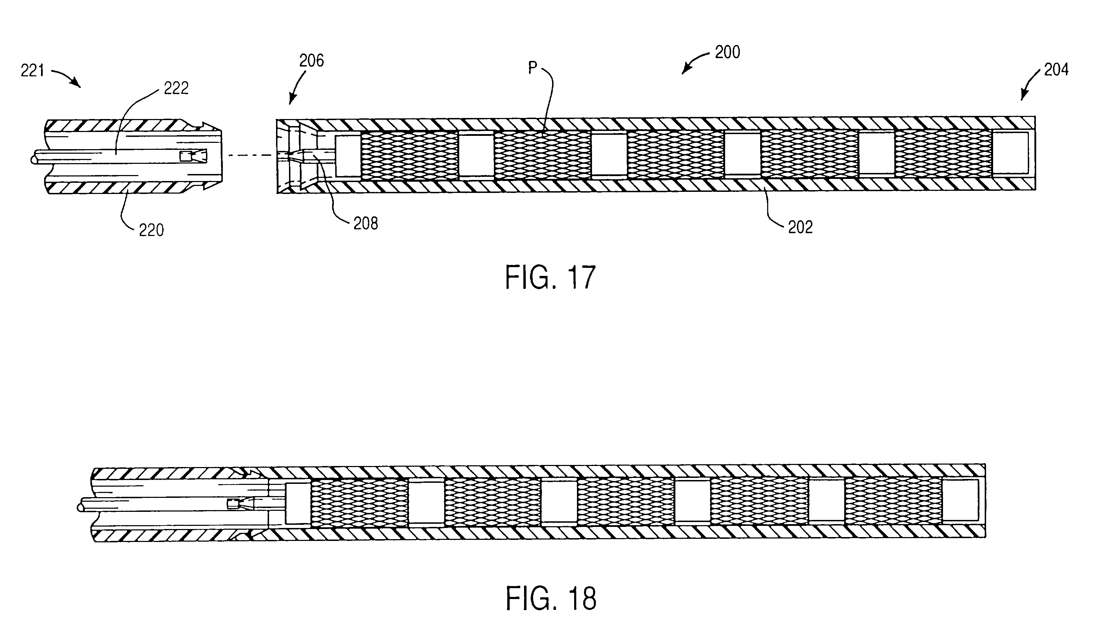

Referring now to Fig. 17 and 18, a prosthesis cartridge 200 comprises a sheath extension 202 having a distal end 204 and a proximal end 206. A prosthesis P is contained within the sheath extension 202 and is mounted over a shaft extension 208. Typically, the prosthesis P will be anchored on the shaft extension 208 using penetrating stays (not shown) as described in connection with previous embodiments. The prosthesis cartridge 200 is releasably connectable to a delivery catheter 221 including a sheath 220 (or other elongate member) and shaft 222. The proximal end of the cartridge sheath 202 is configured to couple to the distal end of the catheter sheath 220. Similarly, the proximal end of the shaft extension 208 is configured to selectively couple to the distal end of the shaft 222. In this way, a user can select the diameter, length, and other characteristics of the prosthesis P which are desired to be employed in a procedure. The prosthesis, which is part of cartridge 200 (and preferably packaged in a separate, sterile pouch or other container) may then be attached to the distal end of the delivery catheter (which is separately packaged in a sterile pouch or other container) having the necessary sheath and shaft connections. The catheter sheath 220 could alternatively comprise other, non-tubular structures (elongate members). It is necessary only that the elongate member be able to connect to the sheath extension 202 to be proximally retracted over the prothesis P (and optionally distally advanced) to effect release and recapture of the prosthesis as described above.

Referring now to Figs. 19A-19D, yet another embodiment of a delivery catheter 250 will be described. Delivery catheter 250 includes flexible shaft 252 having a central lumen for receiving a guide wire GW. A sheath 254 is slidably mounted over the shaft 252, generally as described for previous embodiments. The catheter 250 differs from previous embodiments, however, in the nature of the retaining structure which is used for holding prosthesis P in place on the flexible shaft 252. The retaining structure comprises a distal anchor 256, which is conveniently in the form of a cap or other receptacle which can receive a distal end of the prosthesis therein. A proximal anchor 258 is mounted at the distal end of a sliding tube 260. As shown in Fig. 19A, when the catheter 250 is introduced to blood vessel BV the prosthesis P will be maintained in its collapsed configuration by the anchors 256 and 258, and sheath 254 will cover the prothesis and anchor structures.

After introduction, as illustrated in Fig. 19B, the sheath 254 may be withdrawn proximally to expose the prosthesis P. The prosthesis P, however, remains radially compressed by the anchors 256 and 258, even after the sheath 254 has been fully withdrawn, as illustrated in Fig. 19C. The prosthesis P may be fully released by moving the anchors 256 and 218 axially apart in order to free the compressed ends of the prosthesis, as illustrated in Fig. 19D. Prior to release, however, the exposed prostheses can be carefully positioned without interference from the sheath 254. It is a particular advantage that such partial release is achieved while still being able to readily recapture the prosthesis by readvancing the sheath 254.

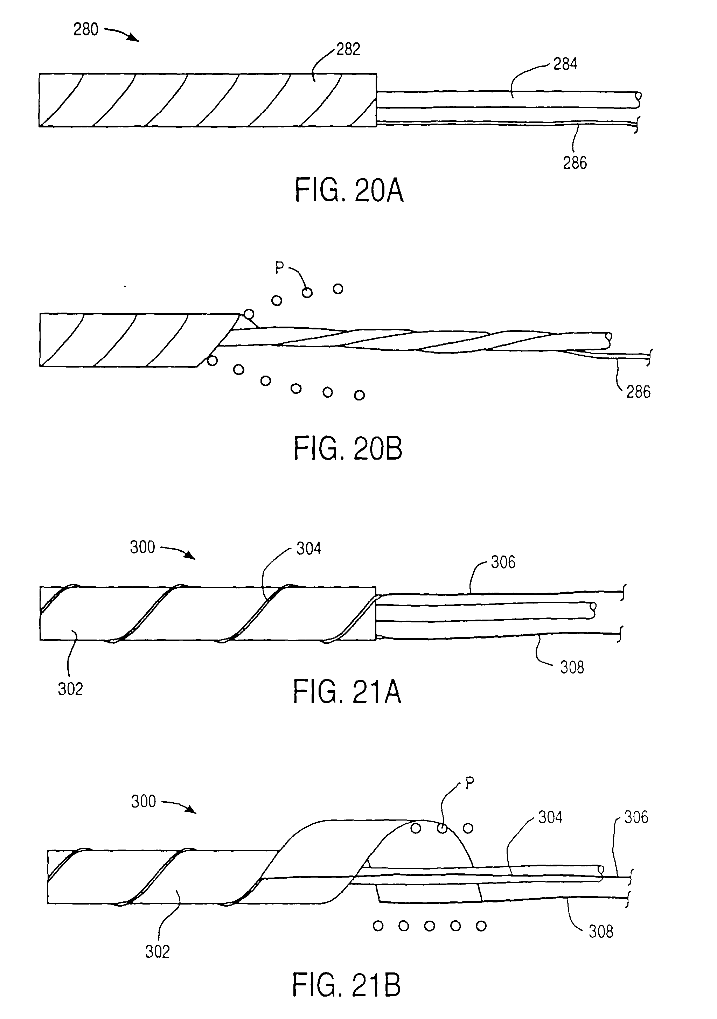

Referring now to Figs. 20A and 20B, an embodiment of a delivery catheter constructed in accordance with the principles of the present invention is illustrated. The retaining structure 280 will fully cover and compress the prostheses P, and will usually be maintained within an outer sheath (not shown) equivalent to the delivery catheter sheaths illustrated previously. The retaining structure 280 will maintain radial compression of the prosthesis P within the sheath, regardless of whether the sheath covers the prosthesis. Thus, the sheath of the associated delivery catheter may be proximally retracted prior to release of the prostheses P.

The retaining structure 280 comprises a helically wound ribbon, which may optionally be formed as a helically scored or perforated cylinder. The retaining structure 280 is mounted on flexible shaft 284, typically with a distal portion of the helical ribbon attached directly or indirectly to the shaft. A pull cord 286 is attached to a proximal end of the helical ribbon, and the ribbon may be withdrawn from over the prostheses P by pulling proximally on the pull cord, as illustrated in Fig. 20b.

Yet another embodiment of the retaining structure is illustrated in Figs. 21A and 21B. Retaining structure 300 comprises a cylinder 302 having a helical wire 304 disposed over its surface. The wire 304, when pulled from the cylinder 302, separates adjacent sections of the cylinder so that they break apart, as illustrated in Fig. 21B. Thus, by attaching a first pull cord 306 to a proximal end of the wire 304, the wire can be withdrawn by pulling proximally. The resulting ribbon-like section of the cylinder may then be withdrawn by pulling on a second pull cord 308, also as shown in Fig. 21B. The prostheses P is thus released from the catheter.

Yet another embodiment of a retaining structure is illustrated in Figs. 22A and 22B structure 320 is a cylinder 322 having a single axial break line 324 formed along one side thereof. It will be appreciated that more than one axial break line may be provided. Only one is illustrated, however, for convenience. A slide structure 326 secured to the cylinder 322 at a distal end of the break line 324. A pull cord 328 is attached to the slide structure 326. Optionally, multiple pull cords could be used. The slide structure 326 may be drawn proximally in order to open the breakline 324 in the manner of a zipper, as illustrated in Fig. 22B. In this way, the prostheses P can be released.

Yet another embodiment of the retaining structure 340 is illustrated in Figs. 23A and 23B. The retaining structure 340 comprises a plurality of individual resilient axial members 342 which are captured at their distal ends and an anchor 344. The axial elements 342 are permanently mounted in a ring structure 346 at the distal end of catheter body 348. The anchor 344 is secured at the distal end of a flexible shaft 350. The axial elements 342 are spring-loaded so that when the anchor 344 is moved distally by advancing the shaft 350, as illustrated in Fig. 23b, the individual elements will spring radially apart at the distal end. In this way, prosthesis P can be released from the retaining structure 340.

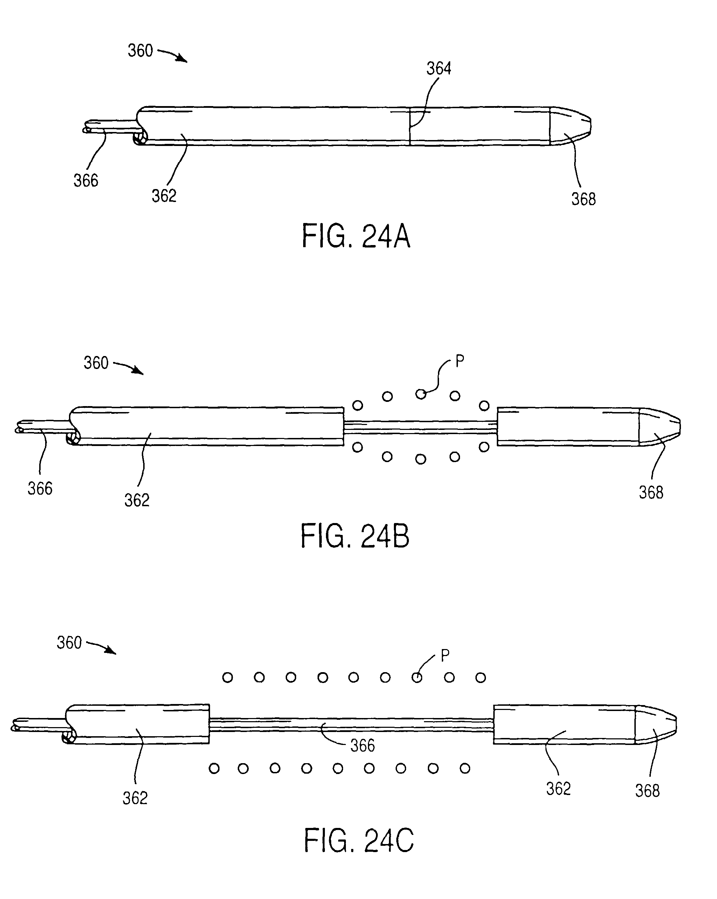

Referring now to Figs. 24A-24C, another embodiment of a retaining structure not falling under the scope of the claims will be described. The retaining structure 360 is a thin-walled tube 362 which is weakened along a circumferential (or helical) line 364, typically in the form of a score, perforation, or the like. Flexible shaft 366 secured to a distal end cap 368. By axially advancing the shaft 366, the end cap 368 and the attached portion of cylinder 362 between the score line 364 and the end cap will be pulled away from the remainder of the cylinder 362. In this way, the prostheses P can be released. The prostheses is first partially released, as shown in Fig. 24B. After the cylinder segments are fully spaced-apart, the prostheses is fully released, as shown in Fig. 24C.

Referring now to Fig. 25, a delivery catheter 430 constructed in accordance with the principles of the present invention comprises a tubular cover 432 and a shaft or inner catheter body 434. Cover 432 has a central lumen 36 extending from a proximal end 438 to a distal end 440. Shaft 434 is slidably received within central lumen 436 and extends proximally of the proximal end of cover 432.

A plurality of runners 442 extend distally from the distal end of shaft 434. Runners 442 line a portion of the inner surface of lumen 436, and slide within the lumen with the shaft. Shaft 434 also has a lumen, in which a core shaft 444 is slidably disposed. Core shaft 444 has a guidewire lumen 446. Guidewire lumen 446 optionally receives an intravascular ultrasound (IVUS) imaging transducer to provide imaging prior to, during, and after deployment of the prosthesis. Nosecone 448 is fixed to the distal end of core shaft 444, and can therefore move independently of runners 442.

Graft 10 is radially compressed and restrained within the plurality of runners 442. In turn, cover 432 prevents runners 442 from expanding outward. Runners 442 are formed from a hard material, and distribute the expansion load of prosthesis 10 over the inner surface of central lumen 436. Advantageously, the prosthesis does not invaginate in the lumen surface, and is thus able to slide relative to the cover in response to a moderate distal force. In the embodiment of Fig. 25, the deploying force is applied proximally against a slider 450 attached to distal end 438 of cover 430, while holding a lure fitting 452 at the distal end of shaft 434. An additional lure adaptor 454 at the distal end of core shaft 444 allows the core shaft to be releasably secured to the shaft 434.

Referring now to Figs. 26A and 26B, loading of graft 10 into the distal end 440 of cover 432 is facilitated by use of runners 442. As seen in Fig. 26A, extending shaft 434 distally allows runners 442 to flex outward. Graft 10 may be inserted between the outward flexed runners and compressed by withdrawing runners 442 and shaft 434 into the distal end 440 of cover 432. Nosecone 448 and core shaft 444 are shown attached to shaft 434 during loading. Alternatively, nosecone 448 may be attached to core shaft 444 after the loading of prosthesis 10. Prosthesis 10 is preferably formed of a heat memory alloy such as Nitinol™. To maintain graft 10 in a compressed state, the loading process may be done in a cold environment, such as that provided by a cold spray, liquid nitrogen, freon, an air vortex, or the like.

Referring now to Figs. 27A through 28, placement of graft 10 within a body lumen 460 begins by positioning catheter 430 at a target location. As illustrated in Fig. 27B, graft 10 is allowed to expand by retracting cover 432, proximally relative to shaft 434 and core shaft 444. As cover 432 is retracted, runners 442 maintain their axial position, sliding over the inner surface of cover 432. Once the graft 10 has fully expanded within body lumen 60, it is axially anchored by expansion against the lumen wall between the runners. Runners 442 may then be retracted proximally with shaft 434 and nosecone 448. The hard surface of runner 442 allows shaft 434 to be retracted smoothly, with little possibility of dragging graft 10 from the target position. The graft cover may also help to reduce friction during deployment. The possibility of dragging the prosthesis is further reduced by retracting nosecone 448 having a tapered proximal end 464 independently from shaft 434, as illustrated in Fig. 28. Finally, it will be recognized that the runners may also be used to help recapture a partially-deployed prosthesis.

Referring now to Fig. 29A, the elements of the present graft delivery catheter will be described. Cover 432 must be strong enough to withstand the expansion force of graft 10 but must also be flexible to allow intravascular atraumatic maneuvering. Cover 432 is optionally formed of a high strength thermoplastic elastomer such as Hytrel™. Alternatively, cover 432 may be formed of a braided reinforced polymer tubing or a linear reinforced tubing, preferably having fibers of a polyamide such as Kevlar™, Spectra™, or the like, embedded to improve tensile strength without reducing flexibility. Preferably, the cover includes a radiopaque contrast medium, e.g., a B₄SO₄ compound, to allow imaging of the placement of catheter 30 within a body lumen using fluoroscopy. Shaft 434 is preferably formed from PEEK, nylon, or the like, to provide column strength. Runners 442 are formed from a high strength biocompatible alloy such as Nitinol™, stainless steel, or a stainless steel alloy. Runners 442 are bonded to shaft 434, preferably being laminated between inner and outer layers of nylon, a thermoplastic elastomer such as Pebax™, or the like. Core shaft 444 is also preferably formed of PEEK. Nosecone 448 may be formed of stainless steel and bonded to the distal end of core shaft 444, or may alternatively be molded of a radiopaque plastic comprising Pebax™, nylon, Hytrel™, or the like. In any case, nosecone 448 preferably includes a radiopaque element, thereby giving an indication of the location of the distal end of graft 10 during fluoroscopically guided prostheses placement. In certain embodiments, core shaft 444 further supports marker ring 466, comprising platinum, barium, or the like, to provide a sharp radiographic contrast. Optionally, distal force imparting structure 467 is bonded to the core shaft to slide the compressed prosthesis distally over the runners.

Referring now to Fig. 29B, a helical shaft 435 provides high column strength with flexibility. Helical shaft 435 is formed from a tightly wound, high strength metal, preferably comprising stainless steel. Helical shaft 435 is easily welded to runners 442, where similar metals are used for both. Alternatively, runners 442 are laminated to helical shaft 435 with inner and/or outer layers of nylon, Pebax™, or the like. A composite cover 433 comprising polymer reinforced tubing having braided or linear Kevlar™, Spectra™, or the like, further enhances flexibility of the delivery catheter of the present invention.

The delivery catheters of the present invention significantly reduce the force required to deploy a prosthesis within a body lumen. Nonetheless, the force required to withdraw cover 432 remains substantial. For this reason, the present invention further provides a housing 470 to be attached to the distal end of shaft 434, as illustrated in Fig. 30. Rotation of handle 472 moves follower 474 along a linear screw. Slider 450 at the proximal end of cover 432 is driven axially by the movement of the follower. Cover 432 is withdrawn during deployment of the prosthesis by articulating handle 472 so as to drive slider 450 toward lure fitting 452 at the proximal end of shaft 434. The force required to withdraw the cover is typically on the order of 1 to 10 lbs., requiring only a modest mechanical advantage. However, a mechanical advantage ratio in the range from 5 to 50, as measured from the linear travel at the outside edge of handle 472 to the linear motion of follower 474, provides a highly controlled deployment. Clearly, a wide variety of mechanical linkages are available to provide such a mechanical advantage. It is particularly advantageous to provide a mechanism which allows manipulation with a single hand, as this leaves the alternate hand free to manipulate the cover relative to an introducer sheath. It will be noted that housing 470 allows independent manipulation of core shaft 444 using second lure fitting 454, as described above regarding Fig. 28.

Referring now to Fig. 31, an alternative cover 480 provides an atraumatic distal end 482 with a reduced nosecone diameter, or, alternatively, no nosecone at the distal end of core shaft 444. Atraumatic cover 480 includes a series of splits 484 to allow the distal tip of atraumatic cover 480 to open during deployment of prosthesis 10.

Referring now to Fig. 32, a further alternative cover 490, having runners 492 embedded within the central lumen, will also reduce the friction between the prosthesis and the cover during prosthesis placement. Furthermore, such a structure eliminates any danger of injury to the walls of a body lumen during placement by a distal movement of the exposed runners. Moreover, similar safety advantages could be obtained using the delivery catheter of Fig. 25 by retaining runners 442 within cover 432 during deployment of prosthesis 10. An alternative structure must be provided to apply a distal force against the prosthesis, such as distal force imparting structure 467 shown in Fig. 29A.

Referring now to Figs. 33A and 33B, alternative cross sections 494 and 496 for a delivery catheter tubular cover or shaft will provide additional column strength without a corresponding increase in stiffness. Slots 495 are also suitable for receiving the runners, thus forming the runner/shaft laminated bond. Indents 497 may receive the free distal portion of the runners to prevent rotation of the prosthesis relative to the cover during manipulation of the shaft. Alternatively, a smooth cover lumen facilitates such rotation by allowing the runners to slidingly rotate against the cover lumen surface.

Referring now to Fig. 34, a brace 590 optionally restrains the prosthesis at the target location while withdrawing cover 432. Brace 590 attaches to introducer sheath 580 with a locking collar 592. Bar 594 extends proximally from locking collar 592, and is slidably received by tabs 596 protruding from housing 470. Once the prosthesis is positioned at the target location, a set screw 598 is tightened to fix the distance between the proximal end of delivery catheter 430 and the introduction sheath 580. Rotating handle 472 thus withdraws cover 430 proximally through introduction sheath 580 without distally advancing shaft 434. This minimizes the danger of advancing the exposed runners into the lumen wall during deployment, and thus allows deployment by a single surgeon. The compressive load on bar 594 is reduced by friction reducer tube 570.

A wide variety of compression bearing structures could be used in place of bar 590. A telescoping tube with single or multiple overlapping sections having set screws would eliminate the protruding proximal end of the rod. Such a telescoping tube may optionally surround catheter 430 between the introducer sheath and housing. Alternatively, a flexible tube having good column stiffness disposed over the delivery catheter also prevents axial movement of the prosthesis, and avoids the long, rigid, and potentially cumbersome bar structure. Such a flexible tube preferably comprises a tightly wound coil analogous to flexible shaft 435 shown in Fig. 29B. Although a fixed length tube may be used, telescoping flexible overlapped tubes, usually having a locking device such as set screws, compressive clamps, or the like, are preferred.

The brace of the present invention may advantageously be used with alternative proximal housings having a wide variety of mechanisms for translating the cover relative to the shaft, including electric motors, foot operated linkages, and the like.

Although the foregoing invention has been described in some detail by way of illustration and example, for purposes of clarity and understanding, certain changes and modifications will be obvious to those of skill in the art. For example, the present cover and/or runners may be attached to the elongate shaft as a cartridge, preferably preloaded with a prosthesis. Thus, the scope of the present invention is limited only by the appended claims.

## Claims

1. A delivery catheter for positioning radially compressible prostheses (P), comprising an elongate flexible shaft (350, 444) having a proximal end and a distal end, a retaining structure (280, 300, 320, 340) near the distal end of the shaft (350, 444) which releasably holds the axial position of the prosthesis (P) on the shaft (350, 444), and a sheath (254, 434) slidably received over the shaft (350, 444) to cover the prosthesis (P) while the prosthesis (P) is held on the shaft (350, 444) by the retaining structure (280, 300, 320, 340) which comprises means (282, 302, 322, 342, 442) for circumferentially supporting the prosthesis (P) within the sheath (254, 434), **characterized** in that the retaining structure (280, 300, 320, 340) is adapted to extend from a proximal end of the prosthesis (P) to the distal end of the prosthesis (P) so that said means (282, 302, 322, 342, 442) for circumferentially supporting the prosthesis (P) supports the prosthesis (P) over its full length.

2. The catheter of claim 1, characterized in that the supporting means comprises a plurality of axial elements (342, 442) attached to the shaft (350).

3. The catheter of claim 2, characterized in that the elements (342, 442) are flexible radially outwardly to release the prosthesis (P).

4. The catheter of claim 3, characterized in that the elements (442) are runners having proximal ends being affixed together.

5. The catheter of claim 4, characterized in that the proximal ends of the runners (442) are affixed to the sheath (434).

6. The catheter of claim 5, characterized in that the supporting means comprises a cover (432) releasably disposed over the elements (442).

7. The catheter of claim 1, characterized in that the supporting means comprises a cover (282, 302, 322) releasably disposed over the prosthesis (P).

8. The catheter of anyone of the claims 1 to 7, characterized in that the retaining structure (280, 300, 320, 340) extends between the prothesis (P) and the surrounding sheath (254, 434).

9. The catheter of anyone of the claims 1 to 8, characterized in that the sheath (254, 434) comprises a flexible polymer material and the retaining structure (280, 300, 320, 340) a material which is harder than that of the sheath (254, 434).

10. The catheter of anyone of the claims 1 to 9, characterized in that a deployable outwardly flarable distal end and means for reconfiguring the distal end of the sheath (254, 434) between a non-flared and a flared configuration are provided.

11. The catheter of anyone of the claims 1 to 10, characterized in that a shaft extension (208) coupling at the distal end of the shaft (222) and a sheath extension (202) coupling at the distal end of the sheath (220) are provided, the prosthesis (P) being radially compressed over the shaft extension (208) and within the sheath extension (202).

## Patentansprüche

1. Einführkatheter zum Positionieren radial komprimierbarer Prothesen (P), mit einem langen, flexiblen Schaft (350, 444), der ein proximales Ende und ein distales Ende besitzt, einer Haltestruktur (280, 300, 320, 340) in der Nähe des distalen Endes des Schafts (350, 444), die die axiale Position der Prothese (P) am Schaft (350, 444) lösbar hält, und einer Hülse (254, 434), die über dem Schaft (350, 444) gleitend aufgenommen ist, um die Prothese (P) zu bedecken, während die Prothese (P) auf dem Schaft (350, 444) durch die Haltestruktur (280, 300, 320, 340) gehalten wird, die eine Einrichtung (282, 302, 322, 342, 442) umfaßt, um die Prothese (P) in der Hülse (254, 434) in Umfangsrichtugn zu unterstützen, **dadurch gekennzeichnet**, daß die Haltestruktur (280, 300, 320, 340) so beschaffen ist, daß sie sich von einem proximalen Ende der Prothese (P) zum distalen Ende der Prothese (P) erstreckt, so daß die Einrichtung (282, 302, 322, 342, 442) zum Stützen der Prothese (P) in Umfangsrichtung die Prothese (P) auf ihrer gesamten Länge stützt.

2. Katheter nach Anspruch 1, dadurch gekennzeichnet, daß die Stützeinrichtung mehrere axiale Elemente (342, 442) umfaßt, die am Schaft (350) befestigt sind.

3. Katheter nach Anspruch 2, dadurch gekennzeichnet, daß die Elemente (342, 442) radial auswärts flexibel sind, um die Prothese (P) freizugeben.

4. Katheter nach Anspruch 3, dadurch gekennzeichnet, daß die Elemente (442) Kufen sind, deren proximale Enden aneinander befestigt sind.

5. Katheter nach Anspruch 4, dadurch gekennzeichnet, daß die proximalen Enden der Kufen (442) an der Hülse (434) befestigt sind.

6. Katheter nach Anspruch 5, dadurch gekennzeichnet, daß die Stützeinrichtung eine Abdeckung (432) aufweist, die über den Elementen (442) lösbar angeordnet ist.

7. Katheter nach Anspruch 1, dadurch gekennzeichnet, daß die Stützeinrichtung eine Abdeckung (282, 302, 322) umfaßt, die über der Prothese (P) lösbar angeordnet ist.

8. Katheter nach einem der Ansprüche 1 bis 7, dadurch gekennzeichnet, daß sich die Haltestruktur (280, 300, 320, 340) zwischen der Prothese (P) und der umgebenden Hülse (254, 434) erstreckt.

9. Katheter nach einem der Ansprüche 1 bis 8, dadurch gekennzeichnet, daß die Hülse (254, 434) ein flexibles Polymermaterial umfaßt und die Haltestruktur (280, 300, 320, 340) ein Material, das härter als dasjenige der Hülse (254, 434) ist, umfaßt.

10. Katheter nach einem der Ansprüche 1 bis 9, dadurch gekennzeichnet, daß ein nach außen enffaltbares, bauschbares distales Ende und eine Einrichtung zum Umkonfigurieren des distalen Endes der Hülse (254, 434) zwischen einer nicht gebauschten und einer gebauschten Konfiguration vorgesehen sind.

11. Katheter nach einem der Ansprüche 1 bis 10, dadurch gekennzeichnet, daß eine Schaftverlängerung (208), die mit dem distalen Ende des Schafts (222) gekoppelt ist, und eine Hülsenverlängerung (202), die mit dem distalen Ende der Hülse (220) gekoppelt ist, vorgesehen sind, wobei die Prothese (P) über der Schaftverlängerung (208) und in der Hülsenverlängerung (202) radial komprimiert ist.

## Revendications

1. Cathéter d'introduction destiné à positionner radialement des prothèses pouvant être compressées (P), comprenant une tige flexible allongée (350, 444) présentant une extrémité proximale et une extrémité distale, une structure de retenue (280, 300, 320, 340) à proximité de l'extrémité distale de la tige (350, 444) qui maintient de manière amovible la position axiale de la prothèse (P) sur la tige (350, 444), et une gaine (254, 434) reçue de manière à pouvoir coulisser sur la tige (350, 444) afin de recouvrir la prothèse (P) lorsque la prothèse (P) est maintenue sur la tige (350, 444) par la structure de retenue (280, 300, 320, 340) qui comprend des moyens (282, 302, 322, 342, 442) destinés à supporter circonférentiellement la prothèse (P) à l'intérieur de la gaine (254, 434), caractérisé en ce que la structure de retenue (280, 300 320, 340) est conçue de manière à s'étendre depuis une extrémité proximale de la prothèse (P) vers l'extrémité distale de la prothèse (P) de telle sorte que lesdits moyens (282, 302, 322, 342, 442) destinés à supporter circonférentiellement la prothèse (P) supportent la prothèse (P) sur la totalité de sa longueur.

2. Cathéter selon la revendication 1, caractérisé en ce que les moyens support comprennent une pluralité d'éléments axiaux (342, 442) fixés sur la tige (350).

3. Cathéter selon la revendication 2, caractérisé en ce que les éléments (342, 442) sont flexibles radialement vers l'extérieur afin de relâcher la prothèse (P).

4. 'Cathéter selon la revendication 3, caractérisé en ce que les éléments (442) sont des broches dont les extrémités proximales sont fixées ensemble.

5. Cathéter selon la revendication 4, caractérisé en ce que les extrémités proximales des broches (442) sont fixées sur la gaine (434).

6. Cathéter selon la revendication 5, caractérisé en ce que les moyens support comprennent un couvercle (432) disposé de manière amovible au-dessus des éléments (442).

7. Cathéter selon la revendication 1, caractérisé en ce que les moyens support comprennent un couvercle (282, 302, 322) disposé de manière amovible au-dessus de la prothèse (P).

8. Cathéter selon l'une quelconque des revendications 1 à 7, caractérisé en ce que la structure de retenue (280, 300, 320, 340) s'étend entre la prothèse (P) et la gaine enveloppe (254, 434).

9. Cathéter selon l'une quelconque des revendications 1 à 8, caractérisé en ce que la gaine (254, 434) est constituée en un matériau polymère souple et la structure de retenue (280, 300, 320, 340) en un matériau qui est plus dur que celui de la gaine (254, 434).

10. Cathéter selon l'une quelconque des revendications 1 à 9, caractérisé en ce qu'une extrémité distale déployable pouvant s'évaser vers l'extérieur et des moyens destinés à modifier la configuration de l'extrémité distale de la gaine (254, 434), entre une configuration non évasée et évasée, sont prévus.

11. Cathéter selon l'une quelconque des revendications 1 à 10, caractérisé en ce qu'une extension de tige (208) pouvant être couplée à l'extrémité distale de la tige (222) et une extension de gaine (202) pouvant être couplée à l'extrémité distale de la gaine (220) sont prévues, la prothèse (P) étant comprimée radialement au-dessus de l'extension de tige (208) et à l'intérieur de l'extension de gaine (202).
